# FASCICULE DE BREVET EUROPEEN

(11) **EP 2 793 798 B1**
(45) Date de publication et mention de la délivrance du brevet: **02.01.2019**
(21) Numéro de dépôt: 12819089.9
(22) Date de dépôt: 18.12.2012
(51) Int. Cl.: A61K 8/00, A61Q 19/02, C12N 15/11, C12Q 1/68, C12N 15/113

(54) **UTILISATION DE MOLECULES DE MICRO-ARN POUR INFLUENCER LA PIGMENTATION DE LA PEAU**
VERWENDUNG VON MIKRORNA-MOLEKÜLE IN DIE BEEINFLUSSUNG VON PIGMENTIERUNG DER HAUT
USE OF MICRORNA MOLECULES IN INFLUENCING PIGMENTATION OF THE SKIN

(30) Priorité: 19.12.2011 FR 1161950
(43) Date de publication de la demande: 29.10.2014
(73) Titulaire: Chanel Parfums Beauté, 92200 Neuilly-sur-Seine (FR); Institut National de la Santé et de la Recherche Médicale (INSERM), 75013 Paris (FR); Institut Curie, 75005 Paris (FR); Centre National de la Recherche Scientifique (C.N.R.S.), 75794 Paris Cedex 16 (FR)
(72) Inventeur: SAINTIGNY, Gaelle, 75019 Paris (FR); MAHE, Christian, 92200 Neuilly sur Seine (FR); LARUE, Lionel, 91440 Bures Sur Yvette (FR); RAMBOW, Florian, 92160 Antony (FR)
(74) Mandataire: Cabinet Plasseraud
(86) Numéro de dépôt international: PCT/FR2012/052977
(87) Numéro de publication internationale: WO 2013/093329

(56) Documents cités:
- WO-A1-2011/063455
- WO-A2-2010/022166
- X. CHEN ET AL: "Epigenetics, MicroRNAs, and Carcinogenesis: Functional Role of MicroRNA-137 in Uveal Melanoma", INVESTIGATIVE OPHTHALMOLOGY & VISUAL SCIENCE, vol. 52, no. 3, 2 mars 2011 (2011-03-02), pages 1193-1199, XP055033845, ISSN: 0146-0404, DOI: 10.1167/iovs.10-5272
- L. T. BEMIS ET AL: "MicroRNA-137 Targets Microphthalmia-Associated Transcription Factor in Melanoma Cell Lines", CANCER RESEARCH, vol. 68, no. 5, 3 mars 2008 (2008-03-03), pages 1362-1368, XP055033767, ISSN: 0008-5472, DOI: 10.1158/0008-5472.CAN-07-2912 cité dans la demande
- DAVID TS WU ET AL: "Mir-434-5p mediates skin whitening and lightening", CLINICAL, COSMETIC AND INVESTIGATIONAL DERMATOLOGY, vol. 1, 7 octobre 2008 (2008-10-07), pages 19-35, XP055033796, cité dans la demande
- LIN SHI-LUNG ET AL: "CHAPTER 4: Recent Application of Intronic MicroRNA Agents in Cosmetics", 2008, CURRENT PERSPECTIVES IN MICRORNAS (MIRNA), SPRINGER NETHERLANDS, NL, PAGE(S) 51 - 72, XP009136584, ISBN: 978-1-4020-8532-1 abrégé page 63, alinéa 1 - page 69, alinéa 1
- MUELLER D W ET AL: "miRNA expression profiling in melanocytes and melanoma cell lines reveals miRNAs associated with formation and progression of malignant melanoma", JOURNAL OF INVESTIGATIVE DERMATOLOGY, NATURE PUBLISHING GROUP, GB, vol. 129, no. 7, 12 février 2009 (2009-02-12), pages 1740-1751, XP002598392, ISSN: 0022-202X, DOI: 10.1038/JID.2008.452 [extrait le 2009-02-12]
- Elsner Peter: "Cosmeceuticals: Drugs vs. Cosmetics", 15 juin 2000 (2000-06-15), Marcel Dekker, Inc., New York, Basel, XP002680865, ISBN: 0824703057 pages 129, 130 pages 133-137

### SEQUENCE LISTING

<110> CHANEL PARFUMS BEAUTE INSERM INSTITUT CURIE CNRS
<120> MICRO-RNA POUR LEUR UTILISATION DANS LA PIGMENTATION
<130> BFF111158
<160> 20
<170> PatentIn version 3.5
<210> 1
   <211> 22
   <212> RNA
   <213> Homo sapiens
<400> 1
   ucucugggcc ugugucuuag gc 22
<210> 2
   <211> 23
   <212> RNA
   <213> Homo sapiens
<400> 2
   uggaagacua gugauuuugu ugu 23
<210> 3
   <211> 23
   <212> RNA
   <213> Homo sapiens
<400> 3
   uuauugcuua agaauacgcg uag 23
<210> 4
   <211> 20
   <212> DNA
   <213> Artificial Sequence
<220>
   <223> primer
<400> 4
   cacgaaccac ggcactgatt 20
<210> 5
   <211> 22
   <212> DNA
   <213> Artificial Sequence
<220>
   <223> primer
<400> 5
   ttttcttgct gccagtctgg ac 22
<210> 6
   <211> 20
   <212> DNA
   <213> Artificial Sequence
<220>
   <223> primer
<400> 6
   tggttccttt tataccactg 20
<210> 7
   <211> 21
   <212> DNA
   <213> Artificial Sequence
<220>
   <223> primer
<400> 7
   cagatccgac tcgcttgttc c 21
<210> 8
   <211> 21
   <212> DNA
   <213> Artificial Sequence
<220>
   <223> primer
<400> 8
   ttgtaacagc accgaggatg g 21
<210> 9
   <211> 22
   <212> DNA
   <213> Artificial Sequence
<220>
   <223> primer
<400> 9
   actgagcgac atcctgtggt tc 22
<210> 10
   <211> 21
   <212> DNA
   <213> Artificial Sequence
<220>
   <223> primer
<400> 10
   gctcatcggc tgttggtatt g 21
<210> 11
   <211> 21
   <212> DNA
   <213> Artificial Sequence
<220>
   <223> primer
<400> 11
   cactttgctg tcccgatgat c 21
<210> 12
   <211> 20
   <212> DNA
   <213> Artificial Sequence
<220>
   <223> primer
<400> 12
   accgtctctc actggattgg 20
<210> 13
   <211> 20
   <212> DNA
   <213> Artificial Sequence
<220>
   <223> primer
<400> 13
   tacttggtgg ggttttcgag 20
<210> 14
   <211> 42
   <212> DNA
   <213> Artificial Sequence
<220>
   <223> primer
<400> 14
   aaactagcgg ccgctgtcca ggttcccaga gaatatctgc tt 42
<210> 15
   <211> 46
   <212> DNA
   <213> Artificial Sequence
<220>
   <223> primer
<400> 15
   ctagaagcag atattctctg ggaacctgga cagcggccgc tagttt 46
<210> 16
   <211> 42
   <212> DNA
   <213> Artificial Sequence
<220>
   <223> primer
<400> 16
   aaactagcgg ccgctgtcca ggttagctgt caatatctgc tt 42
<210> 17
   <211> 46
   <212> DNA
   <213> Artificial Sequence
<220>
   <223> primer
<400> 17
   ctagaagcag atattgacag ctaacctgga cagcggccgc tagttt 46
<210> 18
   <211> 23
   <212> RNA
   <213> Artificial Sequence
<220>
   <223> primer
<400> 18
   aguaaugucc agguucccag aga 23
<210> 19
   <211> 22
   <212> RNA
   <213> Artificial Sequence
<220>
   <223> primer
<400> 19
   cggauucugu guccgggucu cu 22
<210> 20
   <211> 21
   <212> RNA
   <213> Artificial Sequence
<220>
   <223> primer
<400> 20
   aguaaugucc agguuagcgc a 21

## Description

L'invention concerne l'identification et l'utilisation de composés qui inhibent l'expression ou l'activité des micro-RNA pour influencer la pigmentation de la peau, i.e. dépigmenter ou pigmenter la peau.

Les micro-RNA matures (« miRNA », appelés également dans la présente demande « miR ») sont des ARN non codants de petite taille (environ 21 nucléotides de long). Les miRNA ont été montrés comme jouant un rôle en tant que principaux mécanismes de régulation post-transcriptionnelle de l'expression génique. Chez les humains, environ 2000 miRNA ont été décrits (mirBase release 19). Ils semblent jouer un rôle dans le contrôle de l'activité de plus de 60% de tous les gènes codant des protéines, et participent à la régulation de presque tous les processus cellulaires étudiés à ce jour (Friedman et al., 2009). En général, les miRNA inhibent la synthèse protéique par la répression de la traduction et/ou en déstabilisant/dégradant les ARNm. Le miRNA interagit avec l'ARN par une complémentarité de base imparfaite dans sa région "seed" (séquence de 2-8 nucléotides) au niveau du 3'UTR, dans la région de codage (CDS) ou en 5'UTR de l'ARNm (Fabian et al., 2010). Un miRNA mature peut avoir jusqu'à une centaine de cibles ARNm. Un miRNA mature peut être obtenu de différents gènes de miRNA situés sur différents chromosomes dans le génome.

Le processus de pigmentation dans les mélanocytes implique de nombreuses protéines, qui pourraient potentiellement être ciblées par les miRNA. L'importance des miRNA dans le lignage mélanocytaire a été étudiée uniquement dans la genèse du mélanome. Un des derniers exemples publiés est de Chen et al.: les auteurs ont montré que miR-193b réprime la prolifération cellulaire et régule la cycline D1 dans le mélanome (Chen et al., 2010). Il a également été montré que Dicer (une enzyme clé dans la maturation des miRNA) est essentielle pour permettre le développement des mélanoblastes in vivo (Levy et coll., 2010). Ce résultat montre que les miRNA sont importants dans la prolifération - au moins - des mélanoblastes. À ce jour, une seule étude est publiée sur le rôle des miRNA dans le processus de pigmentation (Wu et al., 2008). Les auteurs rapportent que le miR-434-5P réduit la pigmentation, cependant ce miR-434-5P n'a pas été trouvé dans les bases de données publiques humaines. En Août 2012, le miR-145 a un effet dépigmentant sur des mélanocytes humains en ciblant une protéine essentielle au transport des mélanosomes (Dynoodt et al., 2012).

Parmi la grande cohorte des miRNA, on trouve les hsa-mir-330, hsa-mir-7 et le hsa-mir-137.

Le gène du hsa-mir-330 est situé sur le chromosome 19q13.32, et donne lieu à forme mature hsa-miR-330-5p. Le miR-330 agit comme un suppresseur de tumeur dans les cellules de cancer de la prostate en induisant l'apoptose via la suppression médiée par E2F1 de la phosphorylation de AKT (Lee et al., 2009).

Quant au hsa-miR-7 mature, il peut dériver des 3 gènes hsa-mir-7-1 (Chr. 9q21.32), hsa-mir-7-2 (Chr. 15q26.1), et/ou hsa-mir-7-3 (Chr. 19p13.3). Le miR-7 inhibe les voies EGFR et AKT, et est inhibé dans le glioblastome (Kefas et al., 2008). Dans le carcinome de la langue, le miR-7 cible l'IGF1 (Jiang et al., 2010). L'introduction du miR-7 dans des cellules de cancer du sein fortement invasives pourrait inhiber leur motilité, leur pouvoir d'invasion et leur potentiel de formation de tumeurs en réduisant PAK1 (Reddy et al., 2008).

La forme mature du hsa-miR-137 vient du gène du hsa-mir-137 situé sur le Chr. 1p21.3. Le hsa-miR-137 cible MITF (Bemis et al., 2008) et la carboxy-terminal binding protein 1 (CtBP1) dans une lignée cellulaire de mélanome (Deng et al., 2011). En outre, le hsa-miR-137 induit la différenciation des cellules souches de cancer du cerveau (Silber et al., 2008). Par ailleurs, la méthylation du promoteur du hsa-mir-137 est associée avec une faible survie globale des patients atteints de carcinome squameux de la tête et du cou (Langevin et al., 2011).

Il existe donc un besoin de trouver de nouveaux composés utiles pour contrôler la pigmentation cutanée, i.e. pour dépigmenter ou pigmenter la peau.

Notamment, il existe un besoin pour de nouveaux composés dépigmentants.

De manière surprenante, les inventeurs ont identifié que les micro-RNA humains hsa-miR-330-5p, hsa-miR-7 et hsa-miR-137, ont des effets dépigmentants sur les mélanocytes.

La présente invention est définie dans les revendications. La présente invention concerne donc l'utilisation non-thérapeutique d'au moins un micro-RNA choisi parmi hsa-mir-330, hsa-mir-7, leurs formes matures et leurs précurseurs, comme actif dépigmentant.

La présente invention concerne également un micro-RNA choisi parmi hsa-mir-330, hsa-mir-7, leurs formes matures et leurs précurseurs, pour son utilisation non-thérapeutique pour prévenir et/ou traiter les désordres hyperpigmentaires.

De préférence, le micro-RNA choisi parmi hsa-mir-330, hsa-mir-7, leurs formes matures et leurs précurseurs, a une activité dépigmentante lorsqu'il est utilisé à une concentration comprise entre 10⁻⁷ et 10⁻² g/ml.

Par « précurseur de micro-RNA », on entend la ou les forme(s) prémature(s) dudit micro-RNA, et notamment leurs formes en « épingle à cheveu ou boucle » (« stem loop »).

Les micro-RNA selon l'invention sont de préférence d'origine humaine, et sont donc de préférence choisis parmi hsa-mir-330, hsa-mir-7, leurs formes matures et leurs précurseurs.

Hsa-mir-330 est accessible sous le numéro MI0000803 dans la base mirbase.org.

Les formes matures de hsa-mir-330 sont les formes hsa-miR-330-5p (accessible sous le numéro MIMAT0004693 dans la base mirbase.org), hsa-miR-330-3p (accessible sous le numéro MIMAT0000751 dans la base mirbase.org).

De préférence, on utilise la forme mature hsa-miR-330-5p de séquence 5'-UCU CUG GGC CUG UGU CUU AGG C-3' (SEQ ID NO :1).

Par « hsa-miR-7 », on entend les formes hsa-mir-7-1, hsa-mir-7-2 et hsa-mir-7-3, accessibles respectivement sous les numéros MI0000263, MI0000264 et MI0000265 dans la base mirbase.org.

Les formes matures de hsa-mir-7-1 sont les formes hsa-miR-7-5p (accessible sous le numéro MIMAT0000252 dans la base mirbase.org), hsa-miR-7-1-3p (accessible sous le numéro MIMAT0004553 dans la base mirbase.org).

Les formes matures de hsa-mir-7-2 sont les formes hsa-miR-7-5p (accessible sous le numéro MIMAT0000252 dans la base mirbase.org), hsa-miR-7-2-3p (accessible sous le numéro MIMAT0004554 dans la base mirbase.org).

La forme mature de hsa-mir-7-3 est la forme hsa-miR-7-5p (accessible sous le numéro MIMAT0000252 dans la base mirbase.org).

Aussi, de préférence, puisque les hsa-mir-7-1, 7-2 et 7-3 ont une forme mature commune hsa-miR-7-5p, on utilise cette dernière de séquence 5'-UGG AAG ACU AGU GAU UUU GUU GU-3' (SEQ ID NO :2).

Hsa-miR-137 est accessible sous le numéro MI0000454 dans la base mirbase.org.

La forme mature de hsa-mir-137 est la forme hsa-miR-137 (accessible sous le numéro MIMAT0000429 dans la base mirbase.org), de séquence 5'-UUA UUG CUU AAG AAU ACG CGUAG-3' (SEQ ID NO :3).

Les désordres hyperpigmentaires selon l'invention sont de préférence choisis parmi le melasma, le chloasma, les lentigines, le lentigo sénile, les hyperpigmentations irrégulières liées au photovieillissement, les taches de rousseur, les hyperpigmentations post-inflammatoires dues à une abrasion ou à une brûlure ou à une cicatrice ou à une dermatose ou à une allergie de contact, les nevis, les hyperpigmentations à déterminisme génétique, les hyperpigmentations d'origine métabolique ou médicamenteuse, et les mélanomes.

La présente invention a également pour objet une méthode in vitro pour l'identification de composés dépigmentants, comprenant les étapes suivantes:
a. mettre au moins un composé test en contact avec un échantillon de mélanocytes;
b. mesurer l'expression ou l'activité d'au moins un micro-RNA choisi parmi hsa-mir-330, hsa-mir-7, leurs formes matures et leurs précurseurs, dans lesdits mélanocytes;
c. sélectionner les composés pour lesquels au moins 20% d'activation de l'expression ou de l'activité d'au moins un desdits micro-RNA, ses formes matures et précurseurs, est mesurée dans les mélanocytes traités en a. par comparaison avec les mélanocytes non traités.

Selon un premier mode de réalisation, l'étape b. est effectué avant et après l'étape a. Dans ce cas, l'expression ou l'activité dépigmentante du micro-RNA mesurée dans les mélanocytes avant l'étape a. correspond à la valeur de contrôle (c'est à dire les mélanocytes non traités). Ainsi, l'étape c. comprend la sélection des composés pour lesquels une activation d'au moins 20%, de préférence au moins 30%, de préférence au moins 40% de l'expression ou de l'activité d'au moins un micro-RNA est mesurée dans les mélanocytes traités dans a. comparé avec les mêmes mélanocytes avant l'étape a.

Selon un autre mode de réalisation, le procédé comporte une première étape a', de préparation des échantillons de mélanocytes. Ainsi, de préférence, la présente invention concerne une méthode in vitro pour l'identification de composés dépigmentants, comprenant les étapes suivantes :
a'. préparer au moins deux échantillons de mélanocytes;
a. mettre l'un des échantillons en contact avec au moins un composé test, puis
b. mesurer l'expression ou l'activité d'au moins un micro-RNA choisi parmi hsa-mir-330, hsa-mir-7, leurs formes matures et leurs précurseurs, dans lesdits échantillons, et
c. sélectionner les composés pour lesquels au moins 20% d'activation de l'expression ou de l'activité d'au moins un desdits micro-RNA, ses formes matures et précurseurs, est mesurée dans les mélanocytes traités en a. par comparaison avec les mélanocytes non traités.

Dans ce second mode de réalisation, l'expression ou l'activité du micro-RNA ou d'une forme mature ou précurseur, mesurée dans l'échantillon de mélanocytes non soumis à l'étape a., correspond à la valeur de contrôle (c'est à dire les mélanocytes non traités).

Le composé à tester peut être de tout type. Il peut être d'origine naturelle ou avoir été produit par synthèse chimique. Il peut provenir d'une banque de composés chimiques structurellement définis, ou de composés ou substances non caractérisés, ou un mélange de composés. Il peut être choisi notamment parmi des composés naturels, qui comprennent des composés d'origine végétale, comme les plantes. De préférence, les composés à tester sont d'origine végétale, de préférence ils sont choisis parmi les extraits botaniques.

Selon l'étape a., le composé à tester est mis en contact avec un échantillon de mélanocytes.

Selon l'étape b., l'expression et/ou l'activité d'au moins un micro-RNA, ses formes matures ou précurseurs, est mesurée dans les mélanocytes. Par «expression d'un micro-RNA», on entend la quantité de micro-RNA produite. L'expression du micro-RNA s'entend aussi bien lors de la transcription du gène codant ledit micro-RNA, que lors de la maturation dudit micro-RNA. Par «activité d'un micro-RNA», on entend l'activité dépigmentante dudit micro-RNA, i.e. la capacité dudit micro-RNA à inhiber la pigmentation cutanée, c'est-à-dire la capacité dudit micro-RNA à diminuer la quantité de mélanine produite par les mélanocytes.

L'homme de l'art est familier avec les techniques quantitatives ou semi-quantitatives pour détecter l'ARNm qui s'hybride au micro-RNA, et ainsi, pour déterminer l'activité du micro-RNA. Des techniques basées sur l'hybridation de l'ARNm avec des sondes spécifiques de nucléotides sont les plus communs, comme Northern blot, RT-PCR (reverse transcriptase polymerase chain reaction), RT-PCR quantitative (qRT-PCR).

L'homme de l'art est également familier avec les techniques quantitatives ou semi-quantitatives de détection des micro-RNA, ou de l'ARNm qui s'hybride au micro-RNA. En particulier, l'expression du micro-RNA peut être mesurée par PCR en temps réel. L'activité du micro-RNA peut être mesurée par PCR en temps réel sur les cibles ARNm, ou en évaluant le niveau de protéines cibles par western blot. De préférence, l'expression du micro-RNA est mesurée par PCR en temps réel. De préférence, l'activité du micro-RNA est mesurée en quantifiant la quantité de mélanine produite.

L'expression ou l'activité du micro-RNA après traitement avec le composé à tester est ensuite comparée à une valeur de contrôle, c'est à dire une valeur obtenue dans les mêmes mélanocytes avant le traitement, ou une valeur obtenue dans un autre échantillon de mélanocytes qui ne sont pas traités.

Selon l'étape c, les composés utiles sont ceux pour lesquels une activation d'au moins 20%, de préférence au moins 30%, de préférence au moins 40% de l'expression ou de l'activité d'au moins un micro-RNA est mesurée dans les mélanocytes traités par rapport aux mélanocytes non traités. De préférence, l'activation de l'expression ou de l'activité des micro-RNA est d'au moins 50%, de préférence d'au moins 60%.

Les composés sélectionnés par le biais des méthodes de dépistage définies dans l'invention peuvent ensuite être testés sur d'autres modèles in vitro et/ou dans des modèles in vivo pour leurs effets sur la pigmentation cutanée. Les composés utiles selon l'invention sont des activateurs des micro-RNA ciblés.

Selon un autre mode de réalisation, la présente invention se rapporte à une méthode in vitro pour l'identification de composés propigmentants, comprenant les étapes suivantes:
a. mettre au moins un composé test en contact avec un échantillon de mélanocytes;
b. mesurer l'expression ou l'activité d'au moins un inhibiteur de hsa-miR-330-5p, et/ou hsa-miR-7, leurs formes matures, dans lesdits mélanocytes;
c. sélectionner les composés pour lesquels au moins 20% d'inhibition de l'expression ou de l'activité d'au moins un desdits micro-RNA, ses formes matures et précurseurs, est mesurée dans les mélanocytes traités en a. par comparaison avec les mélanocytes non traités.

Dans ce cas, les composés sélectionnés à l'étape c) sont des composés inhibiteurs de micro-RNA.

L'inhibiteur peut être un ADN antisens, un ARN ou un siRNA. De préférence, les inhibiteurs de micro-RNA sont des anti-miR. Les anti-miR sont des inhibiteurs de miR qui inhibent spécifiquement les micro-RNA endogènes. Les anti-miR sont des acides nucléiques simple brin conçus pour se lier spécifiquement à des molécules de micro-RNA et les inhiber. Les anti-miR ont une séquence nucléique complémentaire de la séquence du miR cible. Ces inhibiteurs peuvent être introduits dans les cellules en utilisant la transfection ou l'électroporation, de manière similaire à celle utilisée pour les siRNA. L'utilisation d'anti-miR permet une analyse fonctionnelle du miR par la régulation négative de son activité. Les anti-miR sont commercialement disponibles, ils peuvent par exemple être obtenus par Ambion ou Applied Biosystems.

Les miR, les activateurs de miR ou les inhibiteurs de miR identifiés grâce aux méthodes de dépistage décrites ci-dessus peuvent être formulés dans une composition, en combinaison avec un support physiologiquement acceptable, de préférence un milieu cosmétiquement acceptable, c'est à dire un milieu qui convient pour une utilisation en contact avec la peau humaine, sans aucun risque de toxicité, d'incompatibilité, d'instabilité ou de réaction allergique et surtout qui ne cause pas de sensations d'inconfort (rougeurs, tiraillements, picotements, etc) qui sont inacceptables pour l'utilisateur. Ces compositions peuvent être administrées, par exemple, oralement ou par voie topique. De préférence, la composition est appliquée par voie topique. Par voie orale, la composition peut être sous la forme de comprimés, de gélules, de comprimés dragéifiés, de sirops, de suspensions, solutions, poudres, granulés, d'émulsions, de suspensions de microsphères ou nanosphères ou vésicules lipidiques ou polymériques pour la libération contrôlée. Par voie topique, la composition est plus particulièrement destinée au traitement de la peau et les muqueuses et peuvent être sous forme de pommades, crèmes, laits, pommades, poudres, de tampons imbibés, de solutions, gels, sprays, lotions ou de suspensions. Elle peut également être sous la forme de suspensions de microsphères ou nanosphères ou vésicules lipidiques ou polymériques ou de patches polymériques ou d'hydrogels permettant une libération contrôlée. Cette composition pour application topique peut être sous forme anhydre, sous forme aqueuse ou sous la forme d'une émulsion. La composition pour application topique peut être sous la forme d'une émulsion huile-dans-eau, eau-dans-huile ou multiple (E / H / E ou H / E / H), qui peut éventuellement être des microémulsions ou nano-émulsions, ou sous la forme d'une dispersion aqueuse, une solution, un gel aqueux ou une poudre. Dans une variante préférée, la composition est sous la forme d'un gel, une crème ou une lotion.

Le support physiologiquement acceptable de la composition comprend généralement de l'eau et éventuellement d'autres solvants comme l'éthanol.

Cette composition est de préférence utilisée comme un soin et/ou un produit nettoyant pour la peau du visage et/ou des lésions corporelles, et elle peut être notamment sous la forme d'un liquide, un gel ou une mousse, conditionnée, par exemple, dans un flacon-pompe, un aérosol ou un tube, ou sous forme de crème conditionnée, par exemple, dans un bocal. En variante, elle peut être sous la forme d'un produit de maquillage et en particulier un fond de teint ou une poudre libre ou compacte.

Elle peut comprendre divers adjuvants, tels qu'au moins un composé choisi parmi:
- Les huiles, qui peuvent notamment être choisies parmi les huiles de silicone volatiles ou non volatiles, linéaires ou cycliques, telles que les polydiméthylsiloxanes (diméthicones), polyalkylcyclosiloxanes (cyclométhicones) et polyalkylphénylsiloxanes (phényl diméthicones); les huiles synthétiques telles que les huiles fluorées, les alkyl benzoates et les hydrocarbures ramifiés tels que le polyisobutylène; les huiles végétales et en particulier l'huile de soja ou d'huile de jojoba et des huiles minérales telles que l'huile de vaseline;
- Les cires telles que l'ozokérite, cire de polyéthylène, la cire d'abeille ou de cire de carnauba;
- Les élastomères de silicone obtenus notamment par réaction, en présence d'un catalyseur, d'un polysiloxane contenant au moins un groupe réactif (notamment l'hydrogène ou vinyle) et portant au moins un groupe alkyle (notamment méthyle) ou phényle, en position terminale et/ou latérale, avec un organosilicone tel qu'un polysiloxane organohydrogeno ;
- Les tensioactifs, de préférence les tensioactifs émulsionnants, qu'ils soient non ioniques, anioniques, cationiques ou amphotères, et en particulier les esters d'acide gras et de polyol tels que les esters d'acide gras et de glycérol, les esters d'acide gras de sorbitan, les esters d'acides gras et de polyéthylène glycol et les esters d'acides gras de saccharose; éthers d'alcool gras de polyéthylène glycol; alkylpolyglucosides; polysiloxane-polyéthers modifiés; bétaïne et ses dérivés; polyquaterniums; sels de sulfate d'alkyle; sulfosuccinates; sarcosinates; phosphates d'alkyle et de dialkyle, et leurs sels, et savons d'acides gras;
- Les co-tensioactifs tels que les alcools gras linéaires et en particulier l'alcool cétylique et l'alcool stéarylique;
- Les épaississants et/ou gélifiants, et en particulier réticulés ou non réticulés, homopolymères hydrophiles ou amphiphiles et des copolymères, des AMPS et/ou polymères d'acrylamide et/ou de l'acide acrylique et/ou de sels de l'acide acrylique ou d'esters ; gomme xanthane ou la gomme de guar, les dérivés de cellulose, et les gommes de silicone (diméthiconol);
- Les filtres organiques, tels que les dérivés de dibenzoylméthane, des dérivés d'acide cinnamique, les salicylés, les acides para-amino benzoïques, β,β'-diphényl acrylates, les benzophénones, dérivés de benzylidène, phényle benzimidazoles, les triazines, phényl benzotriazoles et dérivés anthraniliques;
- Les filtres inorganiques, à base d'oxydes minéraux sous forme de pigments enrobés ou non ou nanopigments, et en particulier à base de dioxyde de titane ou l'oxyde de zinc;
- Les colorants;
- Les agents conservateurs;
- Les séquestrants tels que les sels d'EDTA;
- Les parfums;
- Et leurs mélanges, sans que cette liste soit limitative.

Des exemples de ces adjuvants sont particulièrement mentionnées dans le dictionnaire CTFA (International Cosmetic Ingredient Dictionary and Handbook publié par Les cosmétiques, produits de toilette et parfums, 11e édition, 2006), qui décrit une grande variété, sans limitation, des ingrédients cosmétiques et pharmaceutiques généralement utilisés dans l'industrie de soins de la peau, qui sont adaptés pour une utilisation comme ingrédients supplémentaires dans les compositions selon la présente invention.

La composition peut également comprendre au moins un composé avec un effet d'optique, tels que des charges, des pigments, des nacres, des agents tenseurs et des polymères, et leurs mélanges.

Les « charges » sont incolores ou blanches, minérales ou de synthèse, lamellaires ou non lamellaires, et sont adaptées pour donner à la composition de la rigidité et/ou de la douceur, un effet mat et/ou de l'uniformité. Comme charges, on peut notamment citer le talc, le mica, la silice, le kaolin, les poudres de Nylon® telles que Nylon-12 (Orgasol® commercialisé par la société Atochem), les poudres de polyéthylène, les poudres de polyuréthane, les poudres de polystyrène, les poudres de polyester, des microbilles de résine de silicone telles que celles vendues par la société Toshiba sous le nom Tospearl®, l'hydroxyapatite, et des microsphères de silice creuses (Silica Beads® de la société Maprecos).

Le terme «pigments» doit être compris comme des particules blanches ou colorées, minérales ou organiques qui sont insolubles dans le milieu, qui sont destinées à colorer et/ou opacifier la composition. Ils peuvent être de taille usuelle ou nanométrique. Parmi les pigments minéraux, on peut citer le dioxyde de titane, l'oxyde de zirconium, le dioxyde de cérium, l'oxyde de zinc, l'oxyde de fer et l'oxyde de chrome.

Le terme «nacres» doit être compris comme des particules irisées qui réfléchissent la lumière. Parmi les nacres envisageables, on peut citer des ressources naturelles comme la perle, le mica recouvert d'oxyde de titane, d'oxyde de fer, de pigment naturel ou avec le bismuth oxychoride, et le mica titane coloré.

La concentration en masse dans la phase aqueuse de ces charges et/ou pigments et/ou nacres est généralement de 0,1% à 20% et de préférence de 0,2% à 7% en poids par rapport au poids total de la composition.

L '«agent tenseur» doit être compris comme signifiant un composé approprié pour rendre la peau tendue. Par le biais de cet effet de tension, il rend la peau lisse et réduit ou même élimine immédiatement les rides et ridules. Parmi les agents tenseurs, on peut citer notamment les polymères d'origine naturelle. Le terme «polymères d'origine naturelle» signifie les polymères d'origine végétale, les polymères dérivés de téguments, les protéines d'oeuf et les latex d'origine naturelle. Ces polymères sont de préférence hydrophiles. Parmi les polymères d'origine végétale, on peut notamment citer les protéines et hydrolysats de protéines, et plus particulièrement des extraits de céréales, de légumineuses et d'oléagineux, tels que les extraits de maïs, de seigle, de blé, de sarrasin, de sésame, de l'épeautre, de pois, de haricots, des lentilles, du soja et de lupin. Les polymères synthétiques sont généralement sous la forme d'un latex ou un pseudo et peut être de type polycondensat ou obtenus par polymérisation radicalaire. On peut citer en particulier les dispersions de polyester/polyuréthane et de polyéther/polyuréthane. De préférence, l'agent tenseur est un copolymère de PVP/dimethiconyl acrylate et de polyuréthane hydrophile (Aquamere S 2001® par la société HYDROMER).

On peut également utiliser des polymères en solution, en dispersion ou en forme de particules, ce qui réduit la brillance de la peau et unifie le teint. Des exemples qui peuvent être mentionnés comprennent les élastomères de silicone; des particules de résine, et leurs mélanges. Comme exemples d'élastomères de silicone, on peut citer les produits commercialisés sous les nom KSG® par la société Shin-Etsu, sous le nom Trefil®, BY29® ou EPSX® par la société Dow Corning ou sous les noms Gransil® par la société Grant Industries.

La composition utilisée selon l'invention peut également comprendre des agents actifs autres que le micro-RNA ou son activateur, et en particulier au moins un actif choisi parmi:
- les agents qui stimulent la production de facteurs de croissance;
- les agents anti-glycation, les agents qui augmentent la synthèse du collagène ou préviennent sa dégradation (agents anti-collagénase et en particulier les inhibiteurs de métalloprotéases matricielles), les agents qui augmentent la synthèse d'élastine ou préviennent sa dégradation (agents anti-élastase);
- les agents qui stimulent la synthèse de l'intégrine ou des constituants d'adhésion focale, comme la tensine ;
- des agents qui augmentent la synthèse des glycosaminoglycanes ou de protéoglycanes ou qui empêchent leur dégradation (anti-protéoglycanase agents);
- des agents qui augmentent la prolifération des fibroblastes;
- des agents dépigmentants ou anti-pigmentants;
- des antioxydants ou anti-radicaux libres ou des agents anti-pollution, et leurs mélanges, sans que cette liste soit limitative.

Des exemples de tels agents sont notamment:
- des extraits de plantes et en particulier les extraits de Chondrus crispus, de Thermus thermophilus, de Pisum sativum (Proteasyl ® TP LS), de Centella asiatica, de Scenedesmus, de Moringa pterygosperma, d'hamamélis, de Castanea sativa, d'Hibiscus sabdriffa, des Polianthes tuberosa, d'Argania spinosa, d'aloe vera, de Narcisse tarzetta, ou de réglisse;
- une huile essentielle de Citrus aurantium (Neroli);
- les α-hydroxy-acides comme l'acide glycolique, l'acide lactique et l'acide citrique, et esters;
- les β-hydroxy-acides comme l'acide salicylique et ses dérivés;
- des hydrolysats de protéines végétales (en particulier du soja ou de noisette);
- les oligopeptides acylés (notamment commercialisé par la société SEDERMA sous les noms commerciaux Maxilip ®, Matrixyl ® 3000, Biopeptide ® CL ou Biopeptide ® EL);
- des extraits de levure et en particulier de Saccharomyces cerevisiae;
- des extraits d'algues et en particulier de laminaires;
- des vitamines et leurs dérivés tels que l'acide ascorbique, l'ascorbyl glucoside, le magnésium ou le sodium ascorbyl phosphate, le palmitate d'ascorbyle, l'ascorbyl tetraisopalmitate, sorbate d'ascorbyle, le tocophérol, l'acétate de tocophéryle et le sorbate de tocophéryle;
- l'arbutine, l'acide kojique, l'acide ellagique, et leurs mélanges.

En variante ou en plus, la composition utilisée selon l'invention peut comprendre au moins un inhibiteur de l'élastase (anti-élastase), comme un extrait de graines de Pisum sativum qui est notamment commercialisé par la société Laboratoires Sérobiologiques / Cognis France sous le nom Proteasyl TP LS®.

La composition peut également contenir des additifs inertes ou des combinaisons de ces additifs, tels que des agents mouillants, stabilisants, régulateurs d'humidité, régulateurs de pH, les modificateurs de pression osmotique, ou des écrans UV-A et UV-B.

Les exemples suivants illustrent l'invention sans en limiter la portée.

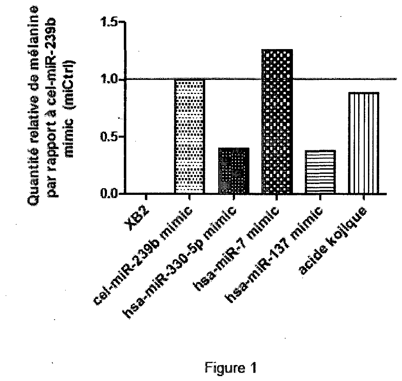

**Figure 1****: Quantité relative de la mélanine dans les lysats de protéines au jour 12 par rapport à miCtrl (cel-miR-239b).**
   L'absorption de mélanine a été mesurée à 470nm. Le lysat de protéines dans les cellules non pigmentées XB2 sert de calibrateur (Abs470nm = 0). L'échantillon miCtrl (cel-miR-239b) est utilisé comme échantillon de référence (Abs470nm = 1). Les taux de mélanine relatifs montrent une réduction des cellules pigmentées MNT-1 traitées avec les mimétiques de hsa-miR-330-5p (mi-330) et hsa-miR-137 (mi-137) par rapport au contrôle de transfection (cel-miR-239b). L'absorption de mélanine est également normalisée par rapport à la quantité de protéines dans chaque échantillon de lysat.

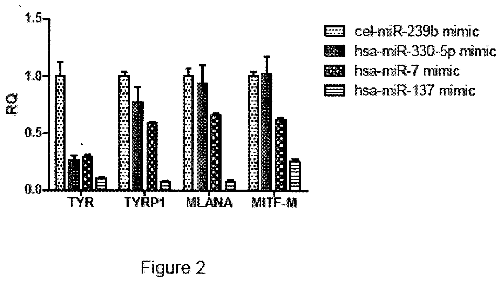

**Figure 2****: expression de l'ARNm de gènes impliqués dans la pigmentation, après traitement par mimétiques de miRNA (jour 12).**
   L'expression des ARNm de Tyr, TYRP1, MLANA et MITF-M a été mesurée par analyse PCR-qRT (méthode de quantification relative ΔΔCt). Les cellules MNT-1 traitées avec le contrôle négatif (cel-miR-239b) ont servi d'échantillon calibrateur (quantité relative, RQ = 1). L'expression de l'ARNm de cellules MNT-1 traitées avec les mimétiques de hsa-miR-330-5p (mi-330), hsa-miR-7 (mi-7) et hsa-miR-137 (mi-137) ont été rapportés comme valeur relative par rapport à l'échantillon calibreur. Les barres d'erreur représentent la déviation standard de la mesure en trois exemplaires. Les niveaux d'ARNm TYR des cellules MNT-1 sont diminués lors de traitements avec des mimétiques de hsa-miR-330-5p, hsa-miR-7 et hsa-miR-137. Les niveaux d'ARNm de TYRP1, MLANA et MITF-M semblent être légèrement réduits par miR-7 et considérablement réduits par miR-137.
**Figure 3** **: expression de miR-330-5p après traitement.**
   L'expression de miR-330-5p est augmentée de plus de 20% dans les cellules MNT-1 après traitement avec le composé 12,085, au bout de 48h par rapport au témoin. Les valeurs sont exprimées comme moyenne, les barres d'erreur représentent l'erreur standard. * P = 0,0233 (t-test de student non aparié)
**Figure 4** **: expression de miR-137 après traitement.**
   L'expression de miR-137 est augmentée de plus de 20% dans les cellules MNT-1 après traitement avec le composé 12,080, au bout de 48h par rapport au témoin. Les valeurs sont exprimées comme moyenne, les barres d'erreur représentent l'erreur standard. * P = 0,0056 (t-test de student non aparié)
**Figure 5** **: expression de miR-7 après traitement.**
   L'expression de miR-7 est augmentée de plus de 20% dans les cellules MNT-1 après traitement avec le composé 12,092, au bout de 48h par rapport au témoin.
**Figure 6** **: efficacité de l'expression de miR-330-5p après traitement.**
   A) Représentation schématique du site de liaison de miR-330-5p dans la partie 3'UTR de la tyrosinase (TYR). La séquence sauvage (WT) du site de liaison de miR-330-5p est indiquée. La séquence mutée (MUT) possède 5 changements nucléotidiques (marqués en rouge).
   B) Le test d'activité du rapporteur luciférase montre la liaison de miR-330-5p à TYR 3'UTR. Des cellules de mélanomes humaines Lu1205 ont été transfectées avec différents vecteurs rapporteurs luciférase (sans insert, WT et MUT) et co-transfectées par des mimétiques de miR-330-5p pour 24h. L'activité luciférase est mesurée sur l'extrait protéique obtenue à partir des cellules lysées. La construction sans insert a servi de référence. Les valeurs sont exprimées sous forme de moyenne. Les barres d'erreur représentent la déviation standard. (** P <0,005 Mann-Whitney test).

### Exemple 1

### Matériels et Méthodes

### Cellules

Des cellules pigmentées de mélanome humain MNT-1 (Reish et al., 1995) ont été cultivées dans du milieu RPMI1640 (# 21875, Gibco), avec 10% de sérum de veau foetal (# 10270, Gibco), 1% de pénicilline-streptomycine (# 15140, Gibco) et 1% de L-glutamine (# 25030, Gibco), à 37°C et 5% de CO2 en atmosphère humide. Des kératinocytes XB2 de souris (Rheinwald et Green, 1975) ont été cultivés dans du DMEM (# 41965, Gibco), avec 10% de sérum de veau foetal (# 10270, Gibco), 1% de pénicilline-streptomycine (# 15140, Gibco) et 1% de L-glutamine (# 25030, Gibco), à 37°C et 5% de CO2 dans une atmosphère humidifiée.

### Mimétiques des miRNA et traitement à l'acide kojique

4x105 de cellules MNT-1 ont été ensemencées par puits dans une plaque à 6 puits (# 353046, Falcon) au jour -1. Au jour 0, les cellules ont été transfectées avec 50 nM de mimétique des miRNA (hsa-mir-137 # C-300604-07-005, hsa-mir-7 # C-300547-05-0005, hsa-mir-330-5p # C-301082-01-0005, contrôle négatif # NC-002000-01-05, Dharmacon), en utilisant 5uL de Lipofectamine 2000 (# 1668-019, Invitrogen). Au jour 1, le milieu a été changé. Au jour 3, les cellules ont été trypsinées et ensemencées à 4x105 cellules par puits. Au jour 4, les cellules ont été transfectées de nouveau avec 50 nM de mimétique des miRNA (voir jour 0). Au jour 5, le milieu a été changé. Au jour 7, les cellules ont été trypsinées et ensemencées à 4x105 cellules par puits. Au jour 8, les cellules ont été transfectées avec 50 nM de mimétique des miRNA (voir jour 0). Au jour 9, le milieu a été changé. Au jour 10, les cellules ont été trypsinées et divisées en deux (d'un à six puits à deux à six puits). Au jour 12, une plaque de six puits a été utilisée pour la lyse de l'ARN et l'autre pour la lyse des protéines. Comme contrôle positif de la dépigmentation, des cellules MNT-1 ont été incubées avec 3,5 mM d'acide kojique (Sigma K3125-5G) pendant 12 jours. Les cellules ont été réparties comme décrit et l'acide kojique a été renouvelé avec le milieu tous les 4 jours. Le pourcentage d'inhibition de l'acide kojique est de l'ordre de 10%.

### Traitement par un inhibiteur de miRNA

Pour étudier l'effet propigmentaire potentiel par inhibition de hsa-miR-7, hsa-miR-137 et hsa-miR-330-5p, 4 x 105 de cellules Lu1205 (une lignée humaine de cellules de mélanome non pigmentées) ont été ensemencées par puits dans une plaque 6 puits (# 353046, Falcon) au jour -1. Au jour 0, les cellules ont été transfectées avec 50 nM d'inhibiteurs respectifs de miRNA (= antagomiR) (hsa-miR-137 # IH-300604-08-0005, hsa-miR-7 # IH-300546-08-0005, hsa-miR-330-5P # IH-301082-02-0005, contrôle négatif # EN-002005-01-05, Dharmacon) en utilisant 5 µL de Lipofectamine 2000 (# 1668-019 Invitrogen). Au jour 1, le milieu a été changé. Au jour 3, les cellules ont été trypsinées et ensemencées à 4 x 105 cellules par puits. Au jour 4, les cellules ont été transfectées à nouveau avec 50 nM d'inhibiteurs de miRNA (voir jour 0). Au jour 5, le milieu a été changé. Au jour 7, les cellules ont été trypsinées et ensemencées à 4 x 105 cellules par puits. Au jour 8, les cellules ont été transfectées avec 50 nM d'inhibiteurs de miRNA (voir jour 0). Au jour 9, le milieu a été changé. Au jour 10, les cellules ont été trypsinées et divisées en deux (d'un à six puits à deux à six puits). Au jour 12, une plaque de six puits a été utilisée pour la lyse de l'ARN et l'autre pour la lyse des protéines.

### Extraction d'ARN total et transcription inverse

700µL de tampon de lyse Qiazol (Qiagen) ont été ajoutés par puits d'une boite de six puits après lavage des cellules deux fois avec du PBS. L'ARN total a été isolé en utilisant le kit miRNeasy Mini (Qiagen) selon le protocole du fabricant. L'ARN total a été élué dans 30 µl d'eau sans RNAse. La concentration d'ARN a été mesurée en utilisant le spectrophotomètre NanoVue (GE Healthcare). 1 µg d'ARN total a été rétrotranscrit en utilisant le système MLV RT-Enzyme (Invitrogen) dans 60µL de réaction pour produire le cDNA correspondant

### qRT-PCR

La PCR en temps réel a été réalisée en utilisant le supermix IQ ™ SYBR ® Green (Biorad) (volume réactionnel final de 25 µL: 23µL de Mix Master et 2µL d'ADNc). Les conditions de thermocyclage (Icycler, Biorad) étaient les suivantes: 95°C pendant 90s, et 40 cycles à 95°C pendant 30s, 60°C pendant 60s et 95°C pendant 10s. Les concentrations en amorces et les séquences sont indiquées dans le tableau 5 suivant :

**Tableau 5: Amorces utilisées pour la qRT-PCR**

| **Gène** | | **Séquence d'amorces** | **Concentration** |
|---|---|---|---|
| **TBP** | Sens | 5'-CAC GAA CCA CGG CAC TGA TT-3' (SEQ ID NO :4) | 300nM |
| | Anti sens | 5'-TTT TCT TGC TGC CAG TCT GGA C-3' (SEQ ID NO :5) | |
| | | | |
| **TYR** | Sens | 5'-TGG TTC CTT TTA TAC CAC TG- 3' (SEQ ID NO :6) | 300nM |
| | Anti sens | 5'-CAG ATC CGA CTC GCT TGT TCC-3' (SEQ ID NO :7) | |
| | | | |
| **TYRP1** | Sens | 5’-TTG TAA CAG CAC CGA GGA TGG-3’ (SEQ ID NO :8) | 300nM |
| | Anti sens | 5’-ACT GAG CGA CAT CCT GTG GTT C-3’ (SEQ ID NO :9) | |
| | | | |
| **MLANA** | Sens | 5’-GCT CAT CGG CTG TTG GTA TTG-3’ (SEQ ID NO :10) | 300nM |
| | Anti sens | 5’-CAC TTT GCT GTC CCG ATG ATC-3’ (SEQ ID NO :11) | |
| | | | |
| **MITF-M** | Sens | 5'-ACC GTC TCT CAC TGG ATT GG - 3' (SEQ ID NO :12) | 300nM |
| | Anti sens | 5’-TAC TTG GTG GGG TTT TCG AG- 3’ (SEQ ID NO :13) | |

La quantité relative a été évaluée en utilisant la méthode comparative ΔΔCt. En bref, cette méthode compare la quantité d'expression du gène cible par rapport à une TBP endogène de contrôle, au sein d'un échantillon pour normaliser l'expression. Au sein d'un groupe d'échantillons, un échantillon approprié est choisi comme échantillon calibrateur (miCtrl). Chaque échantillon est ensuite comparé à une référence désignée pour donner l'expression relative du gène cible par rapport à cet échantillon de référence.

### Mesure de la concentration relative de mélanine

Au jour 12, les cellules ont été lavées dans du PBS deux fois et lysées dans 75µL/puits d'une boite de 6 puits de tampon de lyse des protéines RIPA. Les lysats de protéines ont été transférés dans des tubes Eppendorf de 1.5 ml et vortexé légèrement pour homogénéiser la solution avant de placer le tube sur la glace ou au stockage à -80°C. 2µL de lysat de protéines a été utilisé pour mesurer l'absorbance de la mélanine à 470nm en utilisant le spectrophotomètre NanoVue (GE Healthcare). Comme échantillon calibreur (référence = 0), on a utilisé le lysat protéique des kératinocytes XB2 non pigmentés. Pour détecter la quantité relative de mélanine, tous les échantillons ont été comparés à l'échantillon contrôle de transfection (miCtrl). L'absorbance à 470nm de l'échantillon miCtrl été fixée à 1 comme référence. De plus, l'absorption de mélanine à 470nm a été normalisée en tenant compte de la concentration en protéines de chaque échantillon par rapport à miCtrl.

### Western blot

Les protéines ont été extraites d'une plaque de six puits pendant 30 minutes à 4°C en utilisant 75µL de tampon de radio-immunoprécipitation (RIPA) (1% NP40, 0.5% de désoxycholate de sodium, 0,1% SDS dans du PBS) avec des inhibiteurs de protéase (Roche). Les lysats de protéines ont été centrifugés pendant 30 min à 14000 rpm à 4°C. Le surnageant a été transféré dans un tube Eppendorf 1,5 ml pré-refroidi et placé sur la glace. La concentration en protéines a été déterminée avec le BCA Protein Assay (ThermoFisher). Les protéines (50µg) ont été séparées sur un gel 10% SDS-polyacrylamide et transférées sur des membranes de nitrocellulose ProTron (Whatman) à 100V pendant 1h à température ambiante. Les membranes ont été saturées avec 5 de poudre de lait écrémé dans une solution Tris saline tamponnée (SCT). Les membranes ont été sondées avec l'anticorps primaire dilué (voir ci-dessous) dans 0,5% (v / v) de Tween / SCT (TTBS) pendant la nuit, puis soumises à trois lavages de cinq minutes chacun dans TTBS. Les anticorps secondaires ont été dilués dans du TTBS 1:10,000 et appliqués pendant 1h à température ambiante. Les membranes sont ensuite lavées trois fois dans TTBS pendant 10min chacune et développées avec la solution chimioluminescente SuperSignal WestPico (Thermoscientific) à l'aide d'Amersham Hyperfilm (GE Healthcare).

Les anticorps primaires utilisés sont les suivants :
Polyclonal de chèvre anti-Tyr (C-19), SC-7833 (Santa Cruz), dilué à 1 / 1,000
Polyclonal de chèvre anti-Tyrp1 (G-17), le SC-10443 (Santa Cruz), dilué à 1 / 2,000
Monoclonal de souris anti-MLANA (A103), le SC-20032 (Santa Cruz), dilué à 1 / 500
Polyclonal de lapin anti-MITF (don de S. Saule), dilué à 1 / 1,000
Monoclonal de souris anti-β-actine (CA-15), A5441 (Sigma), 1 / 7,500

Les anticorps secondaires utilisés sont les suivants :
- IgG anti-souris HRP, W402B (Promega), 1 / 10 000
- Anti-IgG de chèvre HRP, 705-035-147 (Jackson ImmunoResearch), 1 / 10 000
- IgG anti-lapin HRP, NA934 (GE Healthcare), 1 / 10 000

### Résultats

### Les hsa-miR-330-5p et hsa-miR-137 affectent les niveaux de pigmentation

Les cellules MNT-1 traitées avec les mimétiques de hsa-miR-330-5p, hsa-miR-137 pendant 12 jours montrent une réduction significative des niveaux de pigmentation globale par rapport aux cellules traitées avec le contrôle négatif (photos non montrées). Les kératinocytes XB2 servent de contrôle pour les cellules non pigmentées. Les photos de lysats protéiques au jour 12 montrent que les cellules MNT-1 traitées avec miR-7 ne montrent pas d'effet significatif sur la pigmentation globale. Comme contrôle positif de la dépigmentation, les cellules MNT-1 ont été traitées avec l'acide kojique.

La quantification des taux relatifs de mélanine, en mesurant l'absorbance à 470nm, a confirmé la réduction de la mélanine dans les cellules MNT-1 traitées avec les mimétiques de hsa-miR-330-5p ou hsa-miR-137 (Fig. 1). Le cel-miR-239b est utilisé comme contrôle de transfection. Au niveau moléculaire, le traitement par mimétique de hsa-miR-330-5p a diminué la teneur en protéines TYR comme le montre l'analyse par Western blot (photos non montrées). De plus hsa-miR-330-5p semble réduire les niveaux d'ARNm TYR (Fig. 2). Le traitement par mimétique de hsa-miR-137 réduit légèrement le niveau de protéine TYRP1 et abolit MLANA et MITF (photos non montrées). Les niveaux d'expression d'ARNm de TYR, TYRP1, MLANA et MITF semblaient être diminués de manière significative par traitement avec le mimétique de hsa-miR-137 (Fig. 2).

De ces résultats, les inventeurs concluent que le traitement des cellules MNT-1 avec des antagomiR dirigés contre hsa-miR-330-5p, hsa-miR-137 et hsa-miR-7 induirait la stabilisation des gènes cibles pigmentaires. Cette stabilisation devrait induire la pigmentation.

### Hsa-miR-7 diminue l'expression des protéines TYR et MITF

Des cellules MNT-1 traitées avec un mimétique de hsa-miR-7 pendant 12 jours ne montrent pas de réduction significative des niveaux de pigmentation globale par rapport aux cellules contrôle négatif (photos non montrées). La quantification des taux de mélanine relatifs, en mesurant l'absorbance à 470nm, suggère une légère augmentation de la mélanine dans les cellules MNT-1 lors de traitement par mimétique de hsa-miR-7 (Fig. 1). Cependant, au niveau moléculaire, le traitement par mimétique de hsa-miR-7 diminue l'expression protéique de TYR et MITF comme le montre l'analyse par Western blot (photos non montrées). De plus miR-7 semble réduire TYR, TYRP1, MLANA et les niveaux d'ARNm MITF-M (Fig. 2).

### Exemple 2

### Matériels et Méthodes

### 1. Composés

Au jour 0 (J0), 150.000 cellules de mélanome humain MNT-1 ont été ensemencées dans des plaques de 24 puits. À J1, les cellules ont été traitées avec les composés suivants en trois exemplaires biologiques pour 48h:

| | | |
|---|---|---|
| 12.080 | H₂0 | 0.08 |
| 12.085 | DMSO | 4.10⁻⁴ |
| 12.092 | DMSO | 16.10⁻⁵ |
| 12.093 | Ethanol | 4.10⁻⁴ |

Où:
12,080 = ascorbyle glucoside ;
12,085 = leucodopachrome, préparé comme décrit dans la demande de brevet WO 2011/033207 ;
12,092 = diacetylresveratryl thioctate, préparé comme décrit dans la demande de brevet WO 2006/134282 ; et
12,093 = ester tranexamique cétylique (TXC).

Les composés ont été fraîchement préparés en solutions mères 10% (v / v) avant l'obtention de la dilution finale dans un milieu RPMI complet (voir les conditions de culture cellulaire). Le milieu contenant les composés a été vortexé vigoureusement, avant d'être appliqué sur les cellules. De même, les cellules MNT-1 ont été traitées avec du DMSO, éthanol et H₂0 comme contrôle en utilisant des quantités appropriées. Après 48h, les cellules sont rinsées avec du PBS et lysées avec du tampon de lyse 700µL Qiazol (Qiagen) avant de les ranger à -80°C. L'ARN total a été extrait en utilisant le kit miRNeasy (Qiagen) et élué dans 30µl de H₂0. La concentration d'ARN et les ratios 260/280nm et 260/230nm ont été mesurés en utilisant la technologie Nanodrop (Thermo Scientific). Un µg d'ARN total a été réverse transcrit en utilisant le système miScriptII (Qiagen) dans un volume total de 20µl. Après la transcription inverse, 80µl de H₂0 ont été ajoutés aux 20 µl d'ADNc. Pour chaque réaction qRT-PCR, 2µl d'ADNc dilué a servi de modèle. La qRT-PCR a été réalisée en double. Le criblage a été effectué deux fois à des dates indépendantes. Pour analyse des données de qRT-PCR, un seuil moyen a été calculé pour l'ensemble des plaques à 96 puits par écran. Des échantillons témoins multiples DMSO, éthanol (EtOH) et H₂0 ont été moyennés et ont servi d'échantillons d'étalonnage pour les composés correspondants (analyse ΔΔCt). Comme gène de référence, SCARNA17 a été utilisé. Les niveaux d'expression de miARN des échantillons contrôle ont été mis à 100%. Une augmentation ou une diminution d'au moins 20% a été considérée comme significative. Enfin, un test-t de student non apparié a été utilisé pour tester si l'augmentation ou la diminution potentielle de l'expression des miARN est significativement supérieure ou inférieure à 20%.

### 2. Test rapporteur de la luciférase

Le site de liaison prédit du miR-330-5p dans le 3'UTR (position 68-75 du 3'UTR de longueur totale 393nt) de TYR (NM_000372) incluant les bases adjacentes a été conçu en utilisant les amorces suivantes, et cloné dans le vecteur rapporteur pmirGLO (Promega) en aval de la région codant la luciférase de luciole et en utilisant les sites Pmel et XbaI.
LL2086: 5'-AAACTAGCGGCCGCTGTCCAGGTT**CCCAGAG**AATATCTGCTT-3' (SEQ ID NO: 14)
LL2087: 5'-CTAGAAGCAGATATTCTCTGGGAACCTGGACAGCGGCCGCTAGTTT-3' (SEQ ID NO: 15)
LL2088: 5'-AAACTAGCGGCCGCTGTCCAGGTT**AGCTGTC**AATATCTGCTT-3' (SEQ ID NO: 16)
LL2089: 5'-CTAGAAGCAGATATTGACAGCTAACCTGGACAGCGGCCGCTAGTTT-3' (SEQ ID NO: 17)

La ligature des amorces LL2086 (sens) et LL2087 (antisens) représente le type sauvage (WT) TYR 3'UTR séquence, alors que la ligature des amorces LL2088 (sens) et LL2089 (antisens) représente la forme mutée du site de liaison du miR-330- Site 5p dans la région 3'UTR TYR (MUT). Toutes les constructions ont été confirmées par séquençage. A J0, 200.000 cellules Lu1205 ont été ensemencées dans des plaques de 12 puits. À J1, les cellules Lu1205 ont été transfectées avec soit 30ng de vecteur pmirGLO vide (sans insert), soit 30ng de construction WT, soit 30ng de construction MUT. Dans le même temps, les cellules ont été co-transfectées avec 50 nM de mimétique de miR-330-5p (Dharmacon) en utilisant la Lipofectamine 2000 (Invitrogen). Quarante-huit heures après la transfection, les cellules ont été récoltées et analysées à la fois par la luciférase de luciole et par la luciférase de Renilla (incluse dans le vecteur pmirGLO) en utilisant le test double luciférase (Promega) et un luminomètre (Microlumatplus LB96V, Berthold Technologies). Six expériences de transfection individuelles (n = 6) ont été effectuées pour les trois conditions. Les valeurs de luciférase de luciole ont été normalisées aux valeurs de luciférase de Renilla. L'activité luciférase relative a été calculée en établissant la condition de non-insertion comme 1.

### Résultats

### 1. Composés

Des cellules de mélanome humaines pigmentées MNT-1 ont été traitées avec les composés et l'expression des miARN (miR-330-5p, miR-137 et miR-7) a été analysée après 48h.

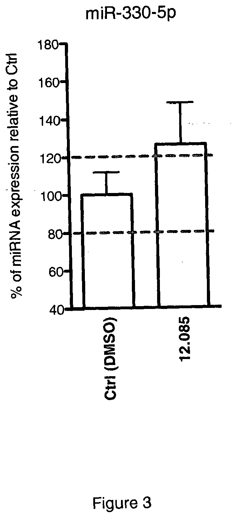

**miR-330-5p.** 12,085 accroît l'expression de miR-330-5p dans des cellules MNT-1 d'au moins 20% par rapport à l'échantillon témoin traité (Figure 3).

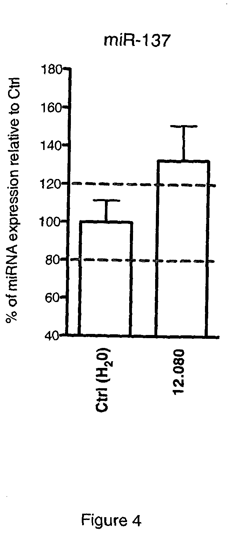

**miR-137.** 12,080 est capable d'induire l'expression de miR-137 de 20% en MNT-1 cellules (Figure 4).

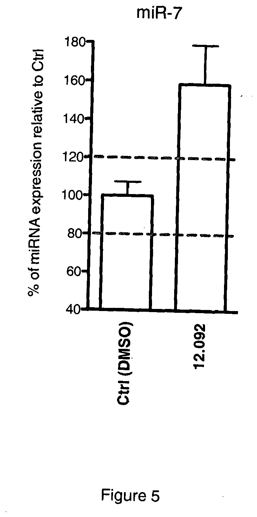

**miR-7.** 12,092 et 12,093 augmentent l'expression de miR-7 dans des cellules MNT-1 d'au moins 20% par rapport à l'échantillon témoin traité (Figure 5).

### 2. Test de luciférase

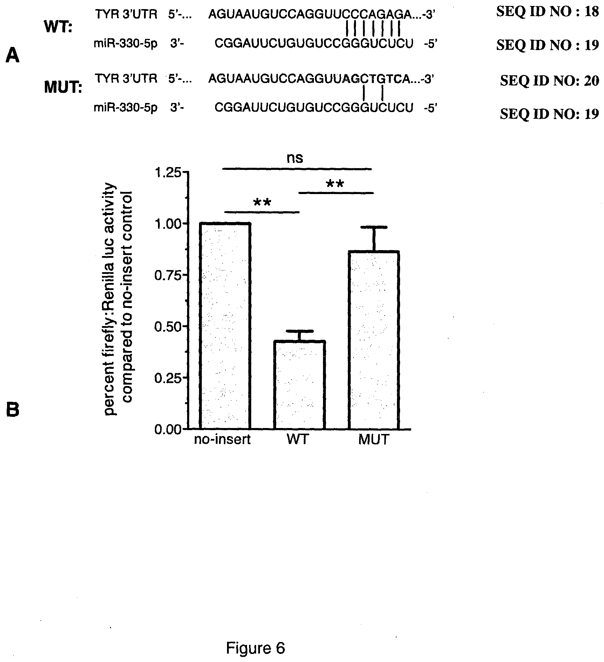

Le site de liaison prédit de miR-330-5p à la 3'UTR de la tyrosinase a été cloné en aval d'un vecteur rapporteur luciférase (WT). De même, une forme mutée du site de liaison de miR-330-5p (MUT) a été cloné (figure 6A).

Mécaniquement, miR-330-5p se lie à son emplacement prévu et provoque la dégradation de l'ARNm correspondant. Cette dégradation de l'ARNm se traduit par une réduction de l'activité luciférase par rapport au contrôle ne possédant pas de cible pour miR-330-5p. Lorsque le site de liaison est muté, le miRNA ne peut plus se lier et la dégradation ne se produit pas.

Les trois constructions ont été transfectées dans des lignées de cellules de mélanome humaines Lu1205, en présence de mimétiques de miR-330-5p. L'activité luciférase a été considérablement réduite pour la construction WT par rapport au témoin sans insert, ce qui suggère que miR-330-5p se lie au site prédictif et induit la dégradation (figure 6B). La forme mutée du site de liaison de miR-330-5p montre une restauration de l'activité luciférase, suggérant que miR-330-5p ne peut plus se lier efficacement à son site prédictif et ne peut donc pas induire de dégradation.

En conclusion, ces résultats montrent que miR-330-5p se lie directement à son site prédit et dégrade l'ARNm de la tyrosinase.

### Références

Bemis LT, Chen R, Amato CM, Classen EH, Robinson SE, Coffey DG, et al. (2008) MicroRNA-137 targets microphthalmia-associated transcription factor in melanoma cell lines. Cancer Res 68:1362-1368.
Chen J, Feilotter HE, Pare GC, Zhang X, Pemberton JG, Garady C, et al. (2010) MicroRNA-193b represses cell proliferation and regulates cyclin D1 in melanoma. Am J Pathol 176:2520-2529.
Deng Y, Deng H, Bi F, Liu J, Bemis LT, Norris D, et al. (2011) MicroRNA-137 targets carboxyl-terminal binding protein 1 in melanoma cell lines. Int J Biol Sci 7:133-137.
Dynoodt P, Mestdagh P, Van Peer G, Vandesompele J, Goossens K, Peelman LJ (2012) Identification of miR-145 as a key regulator of the pigmentary process. J. Invest. Derm. doi: 10.1038/jid.2012.266.
Fabian MR, Sonenberg N, Filipowicz W (2010) Regulation of mRNA translation and stability by microRNAs. Annu Rev Biochem 79:351-379.
Friedman RC, Farh KK, Burge CB, Bartel DP (2009) Most mammalian mRNAs are conserved targets of microRNAs. Genome Res 19:92-105.
Jiang L, Liu X, Chen Z, Jin Y, Heidbreder CE, Kolokythas A, et al. (2010) MicroRNA-7 targets IGF1R (insulin-like growth factor 1 receptor) in tongue squamous cell carcinoma cells. Biochem J 432:199-205.
Kefas B, Godlewski J, Comeau L, Li Y, Abounader R, Hawkinson M, et al. (2008) microRNA-7 inhibits the epidermal growth factor receptor and the Akt pathway and is down-regulated in glioblastoma. Cancer Res 68:3566-3572.
Langevin SM, Stone RA, Bunker CH, Lyons-Weiler MA, Laframboise WA, Kelly L, et al. (2011) MicroRNA-137 promoter methylation is associated with poorer overall survival in patients with squamous cell carcinoma of the head and neck. Cancer 117:1454-1462.
Lee KH, Chen YL, Yeh SD, Hsiao M, Lin JT, Goan YG, et al. (2009) MicroRNA-330 acts as tumor suppressor and induces apoptosis of prostate cancer cells through E2F1-mediated suppression of Akt phosphorylation. Oncogene 28:3360-3370.
Levy C, Khaled M, Robinson KC, Veguilla RA, Chen PH, Yokoyama S, et al. (2010) Lineage-specific transcriptional regulation of DICER by MITF in melanocytes. Cell 141:994-1005.
Reddy SD, Ohshiro K, Rayala SK, Kumar R (2008) MicroRNA-7, a homeobox D10 target, inhibits p21-activated kinase 1 and regulates its functions. Cancer Res 68:8195-8200.
Reish O, Townsend D, Berry SA, Tsai MY, King RA (1995) Tyrosinase inhibition due to interaction of homocyst(e)ine with copper: the mechanism for reversible hypopigmentation in homocystinuria due to cystathionine beta-synthase deficiency. American journal of human genetics 57:127-132.
Rheinwald JG, Green H (1975) Formation of a keratinizing epithelium in culture by a cloned cell line derived from a teratoma. Cell 6:317-330.
Shirdel EA, Xie W, Mak TW, Jurisica I (2011) NAViGaTing the micronome--using multiple microRNA prediction databases to identify signalling pathway-associated microRNAs. PLoS One 6:e17429.
Silber J, Lim DA, Petritsch C, Persson AI, Maunakea AK, Yu M, et al. (2008) miR-124 and miR-137 inhibit proliferation of glioblastoma multiforme cells and induce differentiation of brain tumor stem cells. BMC Med 6:14.
Wu D, Chen JS, Chang DC, Lin SL (2008) Mir-434-5p mediates skin whitening and lightening. Clin Cosmet Investig Dermatol 1:19-35.

## Revendications

1. Utilisation non thérapeutique d'au moins un micro-RNA choisi parmi hsa-miR-330, hsa-miR-7, leurs formes matures et leurs précurseurs, comme actif dépigmentant.

2. Utilisation non-thérapeutique d'un micro-RNA choisi parmi hsa-miR-330, hsa-miR-7, leurs formes matures et leurs précurseurs, dans la prévention et/ou le traitement des désordres hyperpigmentaires, les désordres hyperpigmentaires étant choisis parmi le melasma, le chloasma, les lentigines, le lentigo sénile, les hyperpigmentations irrégulières liées au photovieillissement, les taches de rousseur, les hyperpigmentations post-inflammatoires dues à une abrasion ou à une brûlure ou à une cicatrice ou à une dermatose ou à une allergie de contact, les nevis, les hyperpigmentations à déterminisme génétique et les hyperpigmentations d'origine métabolique ou médicamenteuse.

3. Utilisation selon la revendication 1 ou 2, **caractérisé en ce que** le micro-RNA est choisi parmi hsa-miR-330, hsa-miR-330-5p, hsa-miR-330-3p, hsa-mir-7-1, hsa-miR-7-2 et hsa-miR-7-3, hsa-miR-7-5p, hsa-miR-7-1-3p, hsa-miR-7-2-3pet leurs précurseurs

4. Méthode in vitro pour l'identification de composés dépigmentants, comprenant les étapes suivantes:
a. mettre au moins un composé test en contact avec un échantillon de mélanocytes;
b. mesurer l'expression ou l'activité d'au moins un micro-RNA choisi parmi hsa-mir-330, hsa-mir-7, leurs formes matures et leurs précurseurs, dans lesdits mélanocytes;
c. sélectionner les composés pour lesquels au moins 20% d'activation de l'expression ou de l'activité d'au moins un desdits micro-RNA, ses formes matures et précurseurs, est mesurée dans les mélanocytes traités en a. par comparaison avec les mélanocytes non traités.

5. Méthode selon la revendication 4, **caractérisé en ce que** l'étape b. est effectuée avant et après l'étape a.

6. Méthode selon la revendication 4, **caractérisée** qu'elle comprend les étapes suivantes:
a'. préparer au moins deux échantillons de mélanocytes;
a. mettre l'un des échantillons en contact avec au moins un composé test, puis
b. mesurer l'expression ou l'activité d'au moins un micro-RNA choisi parmi hsa-mir-330, hsa-mir-7, leurs formes matures et leurs précurseurs, dans lesdits échantillons, et
c. sélectionner les composés pour lesquels au moins 20% d'activation de l'expression ou de l'activité d'au moins un desdits micro-RNA, ses formes matures et précurseurs, est mesurée dans les mélanocytes traités en a. par comparaison avec les mélanocytes non traités.

7. Méthode selon l'une quelconque des revendications 4 à 6, **caractérisé en ce que** l'expression ou l'activité d'au moins un micro-RNA choisi parmi hsa-mir-330, hsa-mir-7, ses formes matures et précurseurs, est mesurée par la quantification du micro-RNA correspondant.

8. Méthode selon l'une quelconque des revendications 4 à 7, **caractérisé en ce que** les composés à tester sont choisis à partir d'extraits botaniques.

9. Méthode selon l'une quelconque des revendications 4 à 8, **caractérisé en ce que** les mélanocytes sont issus de lignées de mélanome, de préférence de mélanome humain.

## Patentansprüche

1. Nicht-therapeutische Anwendung wenigstens einer MicroRNA, gewählt aus hsa-miR-330, hsa-miR-7, deren reifen Formen und Vorläufern, als depigmentierender Wirkstoff.

2. Nicht-therapeutische Anwendung einer MicroRNA, gewählt aus hsa-miR-330, hsa-miR-7, deren reifen Formen und Vorläufern, in der Prävention und/oder der Behandlung von Hyperpigmentierungsstörungen, wobei die Hyperpigmentierungsstörungen gewählt sind aus Melasma, Chloasma, Linsenflecken, Altersflecken, unregelmäßigen Hyperpigmentierungen aufgrund von Alterung durch Lichteinwirkung, Sommersprossen, Hyperpigmentierungen als Folge von Entzündungen nach Abschürfungen, Verbrennungen, Narben, Dermatosen oder Kontaktallergien, Muttermalen, Hyperpigmentierungen genetischen Ursprungs und Hyperpigmentierungen metabolischen oder medikamentösen Ursprungs.

3. Anwendung nach Anspruch 1 oder 2, **dadurch gekennzeichnet, dass** die MicroRNA gewählt ist aus hsa-miR-330, hsa-miR-330-5p, hsa-miR-3303p, hsa-miR-7-1, hsa-miR-7-2 und hsa-miR-7-3, hsa-miR-7-5p, hsa-miR-7-1-3p, hsa-miR-7-2-3p und deren Vorläufern.

4. In-vitro-Verfahren zur Identifikation von depigmentierenden Verbindungen, welches die folgenden Schritte umfasst:
a. In-Kontakt-bringen wenigstens einer Testverbindung mit einer Melanozytenprobe;
b. Messen der Expression oder Aktivität wenigstens einer MicroRNA, gewählt aus hsa-miR-330, hsa-miR-7, deren reifen Formen und Vorläufern, in den Melanozyten;
c. Auswählen der Verbindungen, bei welchen wenigstens 20% Aktivierung der Expression oder Aktivität wenigstens einer der MicroRNAs oder deren reifen Formen und Vorläufern in den unter a. behandelten Melanozyten gemessen wird, verglichen mit nicht behandelten Melanozyten.

5. Verfahren nach Anspruch 4, **dadurch gekennzeichnet, dass** Schritt b. vor und nach Schritt a. durchgeführt wird.

6. Verfahren nach Anspruch 4, **dadurch gekennzeichnet, dass** es die folgenden Schritte umfasst:
a'. Zubereiten wenigstens zweier Melanozytenproben;
a. In-Kontakt-bringen einer der Proben mit wenigstens einer Testverbindung, dann
b. Messen der Expression oder Aktivität wenigstens einer MicroRNA, gewählt aus hsa-miR-330, hsa-miR-7, deren reifen Formen und Vorläufern, in den Proben, und
c. Auswählen der Verbindungen, bei welchen wenigstens 20% Aktivierung der Expression oder Aktivität wenigstens einer der MicroRNAs oder deren reifen Formen und Vorläufern in den unter a. behandelten Melanozyten gemessen wird, verglichen mit nicht behandelten Melanozyten.

7. Verfahren nach einem der Ansprüche 4 bis 6, **dadurch gekennzeichnet, dass** die Expression oder Aktivität wenigstens einer MicroRNA, gewählt aus hsa-miR-330, hsa-miR-7, deren reifen Formen und Vorläufern, durch die Quantifizierung der entsprechenden MicroRNA gemessen wird.

8. Verfahren nach einem der Ansprüche 4 bis 7, **dadurch gekennzeichnet, dass** die zu testenden Verbindungen ausgehend von botanischen Extrakten gewählt werden.

9. Verfahren nach einem der Ansprüche 4 bis 8, **dadurch gekennzeichnet, dass** die Melanozyten aus Melanomzelllinien stammen, bevorzugt des menschlichen Melanoms.

## Claims

1. Non-therapeutic use of at least one micro-RNA selected from hsa-miR-330, hsa-miR-7, the mature forms thereof, and the precursors thereof, as an active depigmenting ingredient.

2. Non-therapeutic use of a micro-RNA selected from hsa-miR-330, hsa-miR-7, the mature forms thereof, and the precursors thereof, in the prevention and/or treatment of hyperpigmentation disorders, the hyperpigmentation disorders being selected from melasma, chloasma, lentigines, senile lentigo, irregular hyperpigmentations linked to photoageing, freckles, post-inflammatory hyperpigmentations due to an abrasion or to a burn or to a scar or to a dermatosis or to a contact allergy, nevis, hyperpigmentations with genetic determinism and hyperpigmentations of metabolic or drug origin.

3. Use according to claim 1 or 2, **characterised in that** the micro-RNA is selected from hsa-miR-330, hsa-miR-330-5p, hsa-miR-330-3p, hsa-mir-7-1, hsa-miR-7-2 and hsa-miR-7-3, hsa-miR-7-5p, hsa-miR-7-1-3p, hsa-miR-7-2-3p and the precursors thereof.

4. *In vitro* method for identifying depigmenting compounds, comprising the following steps:
a. placing at least one test compound in contact with a sample of melanocytes;
b. measuring the expression or activity of at least one micro-RNA selected from hsa-mir-330, hsa-mir-7, the mature forms thereof, and the precursors thereof, in said melanocytes;
c. selecting the compounds for which at least 20% activation of the expression or of the activity of at least one of said micro-RNA, the mature and precursor forms thereof, is measured in the melanocytes treated in a. by means of comparison with the untreated melanocytes.

5. Method according to claim 4, **characterised in that** step b. is carried out before and after step a.

6. Method according to claim 4, **characterised in that** it comprises the following steps:
a'. preparing at least two samples of melanocytes;
a. placing one of the samples in contact with at least one test compound, then
b. measuring the expression or activity of at least one micro-RNA selected from hsa-mir-330, hsa-mir-7, the mature forms thereof, and the precursors thereof, in said samples, and
c. selecting the compounds for which at least 20% activation of the expression or of the activity of at least one of said micro-RNA, the mature and precursor forms thereof, is measured in the melanocytes treated in a. by means of comparison with the untreated melanocytes.

7. Method according to any one of claims 4 to 6, **characterised in that** the expression or the activity of at least one micro-RNA selected from hsa-mir-330, hsa-mir-7, the mature and precursor forms thereof, is measured by the quantification of the corresponding micro-RNA.

8. Method according to any one of claims 4 to 7, **characterised in that** the compounds to be tested are selected from botanical extracts.

9. Method according to any one of claims 4 to 8, **characterised in that** the melanocytes are from melanoma lines, preferably human melanoma.
